# EUROPÄISCHE PATENTSCHRIFT

(11) **EP 2 491 998 B1**
(45) Veröffentlichungstag und Bekanntmachung des Hinweises auf die Patenterteilung: **03.06.2015**
(21) Anmeldenummer: 12169453.3
(22) Anmeldetag: 28.06.2007
(51) Int. Cl.: B01D 53/62, B01D 53/86, C01B 3/04, C01B 31/18, C07C 29/151, C10G 2/00, C25B 1/04

(54) **Verfahren zur Wiederaufbereitung von Verbrennungsprodukten fossiler Brennstoffe**
Method for recycling combustion products of fossil fuels
Procédé de recyclage de de produits de combustion de combustibles fossiles

(30) Priorität: 31.07.2006 DE 102006035893
(43) Veröffentlichungstag der Anmeldung: 29.08.2012
(62) Teilanmeldung aus: 07764897.0
(73) Patentinhaber: SunFire GmbH, 28217 Bremen (DE)
(72) Erfinder: Wolf, Bodo, 87719 Mindelheim (DE)
(74) Vertreter: Hansen, Jochen

(56) Entgegenhaltungen:
- WO-A-2006/099573
- US-A- 5 312 843
- MIGNARD D ET AL: "Methanol synthesis from flue-gas CO2 and renewable electricity: a feasibility study", INTERNATIONAL JOURNAL OF HYDROGEN ENERGY, ELSEVIER SCIENCE PUBLISHERS B.V., BARKING, GB, Bd. 28, Nr. 4, 1. April 2003 (2003-04-01), Seiten 455-464, XP004401404, ISSN: 0360-3199, DOI: 10.1016/S0360-3199(02)00082-4

## Beschreibung

Die Erfindung betrifft ein Verfahren zur Wiederaufarbereitung der Verbrennungsprodukte Kohlendioxid und Wasser, wie sie in den Abgasen aus Verbrennungsprozessen oder in der Umgebung vorkommen, in erneuerbare synthetische Brenn- und Kraftstoffe durch elektrische Energie, die nicht mit Hilfe fossiler Brennstoffe erzeugt wurde.

Das Anwendungsgebiet der Erfindung ist die globale, regionale und lokale Versorgung von Industrie, Gewerbe, Kommunen und Bauwerken mit regenerativer Energie in Form stofflich gebundener chemischer Energie.

Beim Stand der Technik galt bis vor kurzem die Verbrennung fossiler Brennstoffe als irreversibler Prozess, d. h. , die Verbrennung ist ein Prozess, der nur in die Richtung der Verbrennungsprodukte verläuft. Daraus wurde in der Praxis die Schlussfolgerung gezogen, dass es nicht möglich bzw. unsinnig ist aus den Verbrennungsprodukten wieder Brennstoffe in der Art der fossilen Brennstoffe, also Kohlenwasserstoffe herzustellen. Die wissenschaftliche Meinungsbildung resultiert aus der Ansiedlung der thermodynamischen Lehre im Bilanzkrei Erde. Diese Lehre wird mit der Veröffentlichung "Öl aus Sonne, die Brennstoffformel der Erde", Ponte Press Verlag GmbH, ISBN 3-920328-49-3 widerlegt. Hier wird beschrieben, dass der unter Zuführung von Sauerstoff exotherm ablaufende Verbrennungsprozess fossiler Brennstoffe Bestandteil der natürlichen reversiblen Stoffumwandlung ist, die sich durch Wiedereinkopplung von Energie umkehren lässt.

Die Druckschrift WO 2006/099 573 A1 offenbart ein Verfahren und eine Anordnung zur Herstellung von synthetischen Kraftstoffen, unterstützt durch die Zufuhr von elektrischer Energie, bevorzugt Energie aus nicht fossilen Energieträgern, sondern vielmehr aus regenerativer Energie. Das Herstellungsverfahren umfasst die Elektrolyse von Wasser und die Verwendung des daraus gewonnenen Wasserstoffes in Kombination mit Kohlendioxid zur Herstellung eines Synthesegases, das zu den synthetisch hergestellten Kraftstoffen, wie Diesel, Benzin oder Kerosin und dgl., prozessiert wird. Der Wasserstoff wird zur Reduktion des Kohlendioxids zu Kohlenmonoxid verwendet, wobei das Kohlenmonoxid weiter mit Wasserstoff in einer Fischer-Tropsch-Synthese zu den o.g. synthetischen Kraftstoffen prozessiert wird. Weiter wird die Energierückgewinnung bzw. Wärmenutzung der einzelnen Prozesse detailiert beschrieben, so dass Abwärme von Prozessen für die Wärmezufuhr anderer Prozesse genutzt werden kann, wobei zur Prozesswärmeübertragung die Verwendung von Wärmetauschern vorgeschlagen ist.

Aus der Druckschrift US 5,312,843 ist ein Verfahren zur Herstellung von Methanol aus Kohlendioxid unter Verwendung der reversen Wassergas-Shift-Reaktion, wobei die Überschusswärme eines Hochtemperatur-gasgekühlten Nuklearreaktors verwendet wird. Hierzu wird Wasserstoff in einer Hochtemperatur-Elektrolyse aus Wasser gewonnen, wobei das Wasser zuvor mit Hilfe von Wärmetauschern und der zugeführten Wärme aus dem Hochtemperatur-gasgekühlten Nuklearreaktor erhitzt wurde. Das Kohlendioxid wird unter Verwendung des gewonnenen Wasserstoffes und unter Anwendung von Wärme zu Kohlenmonoxid reduziert und im Anschluss einer MethanolSynthese zugeführt.

Aus dem Artikel "Methanol synthesis from flue-gas CO2 and renewable electricity: a feasibility study", erschienen im International Journal of Hydrogen Energy 28 (2003) 455-464 ist ein Wiederaufbereitungsverfahren con Kohlenstoffdioxid und Wasser zu Methanol offenbart, wobei eine Elektrolyse zur Erzeugung von Wasserstoff, das Erzeugen eines Syntheserohgases, eine Methanolsynthese und eine Abwärmenutzung entnehmbar sind.

Die technische Aufgabe der Erfindung besteht deshalb darin eine technische Lösung vorzuschlagen, mit der der Vorgang der Verbrennung umgekehrt werden kann.

Die technische Aufgabe wird gelöst durch Abtrennung des im Verbrennungsprozess an Kohlenstoff und Wasserstoff chemisch gebundenen Sauerstoff aus den Verbrennungsprodukten Kohlendioxid und Wasser unter Einkopplung von elektrischer Energie, die vorrangig mit Hilfe regenerativer Energieträger, aber nicht mit Hilfe von fossilen Brennstoffen, hergestellt wurde, indem erfindungsgemäß durch Elektrolyse von Wasser, oder vorzugsweise Wasserdampf, hergestellter Wasserstoff mit Kohlendioxid zu einem Kohlendioxid-Wasserstoff-Gemisch bis zu einem Molverhältnis von 1 zu 3,5 vermischt, dieses in einem Hochtemperaturrekuperator vorgewärmt und danach in einer elektrisch beheizten Vorrichtung oder einem elektrischen Plasmagenerator auf 800 bis 5000°C erhitzt wird, das sich bildende Syntheserohgas rekuperativ zur Vorwärmung des Kohlendioxid-Wasserstoff-Gemisches genutzt, danach unter Abscheidung des Reaktionswassers direkt gekühlt und das dann vorliegende Kohlenmonoxid-Kohlendioxid-Wasserstoff-Gemisch einer Fischer-Tropsch- oder Methanolsynthese zugefahren und dort in die Produkte Kohlenwasserstoffe bzw. Methanol umgewandelt wird, die unter Abscheidung von Wasser gekühlt und erforderlichenfalls kondensiert werden.

Es ist auch erfindungsgemäß, dass die rekuperative Vorwärmung des Kohlendioxid-Wasserstoff-Gemisches und seine weitere Erhitzung durch Zuführung von elektrischer Energie im Beisein von Katalysatoren erfolgt.

Weiterhin erfindungsgemäß ist, das bei der Gas- und Produktkühlung anfallende Wasser gemeinsam mit externem Wasser einzusetzen für die direkte Kühlung des Syntheserohgases und der Syntheseprozesse, dieses dabei zu verdampfen und den Dampf in der Elektrolyse in Wasserstoff und Sauerstoff aufzuspalten.

Der wirtschaftliche Vorteil der Erfindung liegt in der Umwandlung von regenerativer Energie in gegen fossile Brennstoffe vollständig austauschbare erneuerbare Brennstoffe, die über die vorhandene Infrastruktur verteilt werden können und mit denen auf Grund ihrer Speicherfähigkeit unter Umgebungsdruck und -temperatur und der für Menschen unerschöpflichen regenerativen Energiequellen jederzeit eine bedarfsgerechte Versorgung mit Energieträgern und chemischen Einsatzstoffen unabhängig von den fossilen Brennstoffen gesichert werden kann. Gegenüber dem propagierten Wasserstoffsystem hat die Anwendung der Erfindung insbesondere den wirtschaftlichen Vorteil, dass vorhandenen Energiesysteme, die fixes Kapital von mehreren Billionen Euro verkörpern, weiter betrieben werden können und Investitionen in gleicher Größenordnung für den Aufbau einer neuen, wasserstoffgerechten Infrastruktur vermieden werden. Die Erfindung schafft die Grundlagen für den gleitenden Übergang von einer fossilen zu einer solaren Stoff- und Energiewirtschaft.

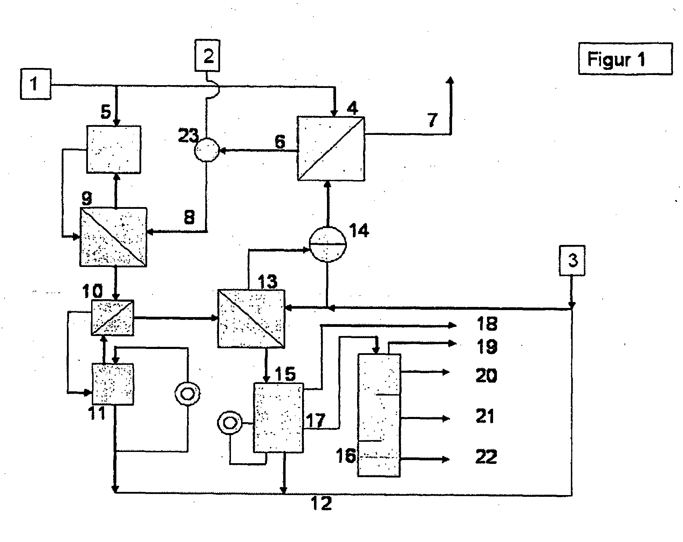

Anhand der beiliegenden Figur wird die Erfindung in einem Ausführungsbeispiel näher erläutert.

### Ausführungsbeispiel

Erfindungsgemäß werden Kohlendioxid und Wasser mithilfe von regenerativer Energie, die in Elektroenergie umgewandelt wurde, in Alkohol oder Kohlenwasserstoffe wie Methan, Benzin, Diesel und Wachs umgewandelt. Zu diesem Zwecke werden dem Prozess über 1 Elektroenergie, über 2 Kohlendioxid und über 3 Wasser zugeführt. Das Wasser wird zusammen mit in der direkten Gaskühlung 11 und der Produktkühlung 15 anfallendem Wasserkondensat, das über den Kondensatsammler 12 gesammelt wird, in der Kühlung 14 des Synthesereaktors 13, vorzugsweise unter einem Druck von 20 bar, verdampft und der Wasserdampf in der Elektrolyse 4 in Wasserstoff 6 und Sauerstoff 7 zerlegt.

Der Wasserstoff 6 wird mit dem über 2 zugeführten Kohlendioxid in einem Molverhältnis von vorzugsweise 3 :1 in 23 unter einem Druck von 19 bar gemischt und das Kohlendioxid-Wasserstoff-Gemisch 8 im Hochtemperaturrekuperator 9 im Gegenstrom zum Syntheserohgas, das aus der elektrisch beheizten Vorrichtung 5 mit einer Temperatur von über 900°C zugeführt wird, gegebenenfalls im Beisein eines Katalysators auf 600 bis 800°C vorgewärmt. Das vorgewärmte Gasgemisch 8 wird in die elektrisch beheizte Vorrichtung 5 geleitet und dort auf mindestens 900°C, im Beisein eines Katalysators, erhitzt. Durch die Energiezufuhr in der elektrisch beheizten Vorrichtung 5 setzt sich das Kohlendioxid-Wasserstoff-Gemisch 8 in ein Syntheserohgas mit einem Anteil Kohlenmonoxid und Wasserstoff von zusammen annähernd 65 Volumen % um. Der Rest ist Wasserdampf und unzersetztes Kohlendioxid. Das Syntheserohgas wird im Rekuperator 10 und durch direkte Kühlung in 11 mit kaltem Wasser, unter Kondensation und Abscheidung von Reaktionswasser auf unter 50°C gekühlt und danach im Rekuperator 10 wieder erwärmt, bevor es nun als Synthesegas dem Synthesereaktor 13 zugeführt wird. Das Synthesegas wird im Syntheserektor 13 je nach Produktionsziel und damit eingebrachtem Katalysator in methanhaltiges Gas 18 und/oder Methanol oder flüssige Kohlenwasserstoffe (Benzin, Kerosin, Diesel) oder Wachs umgewandelt.

Im Falle der Produktion von Methanol und von flüssigen Kohlenwasserstoffen erfolgt die Kondensation der Produkte und die Abtrennung des Produktwassers durch Kühlung im Produktsammelbehälter 15. Bei der Produktion von flüssigen Kohlenwasserstoffen erfolgt die Stofftrennung unter Abscheidung von Entspannungsgas 19 unter leichtem Überdruck in Benzin 20, Diesel 21 und Wachs 22 in der Destillation 16. Die Überleitung des entwässerten Produktes vom Produktsammelbehälter 15 in die Destillation 16 und die Druckreduzierung erfolgt über 17.

### Bezugszeichenliste

- 1: Elektroenergie
- 2: Kohlendioxid
- 3: Wasser
- 4: Elektrolyse
- 5: elektrisch beheizte Vorrichtung (900°C bis 5000°C)
- 6: Wasserstoff (H₂)
- 7: Sauerstoff (O₂)
- 8: Kohlendioxid-Wasserstoff-Gemisch CO₂ + H₂
- 9: Hochtemperatur-Rekuperator
- 10: Rekuperator
- 11: Gaskühlung
- 12: Kondensatsammler
- 13: Synthesereaktor
- 14: Kühlung
- 15: Produktkühlung im Produktsammelbehälter
- 16: Destillation
- 17: Druckreduzierung
- 18: methanhaltiges Gas
- 19: Entspannungsgas
- 20: Benzin
- 21: Diesel
- 22: Wachs
- 23: (Mischer CO₂ + H₂ )

## Patentansprüche

1. Verfahren zur Wiederaufbereitung der Verbrennungsprodukte Kohlendioxid (2) und Wasser (3) unter Einkopplung von elektrischer Energie, die vorrangig mit Hilfe regenerativer Energieträger, aber nicht mit Hilfe fossiler Brennstoffe hergestellt wurde in erneuerbare synthetische Brenn- und Kraftstoffe (18, 20, 21, 22),
wobei eine Elektrolyse (4) von Wasser (3), vorzugsweise Wasserdampf, betrieben wird, der gebildete Wasserstoff (6) mit Kohlendioxid (2) zu einem Kohlendioxid-Wasserstoff-Gemisch (8) bis zu einem Molverhältnis von 1 zu 3,5 vermischt wird, das Kohlendioxid-Wasserstoff-Gemisch (8) in einem Hochtemperaturrekuperator (9) vorgewärmt und danach in einer elektrisch beheizten Vorrichtung (5) oder einem elektrischen Plasmagenerator (5) auf 800 °C bis 5000 °C erhitzt wird,
das so sich bildende Syntheserohgas rekuperativ zur Vorwärmung des Kohlendioxid-Wasserstoffgemisches genutzt, danach in einer Gaskühlung (11) unter Abscheidung von Wasser direkt abgekühlt wird, wobei sich ein Synthesegas ausbildet, das im Weiteren einer Fischer-Tropsch- oder Methanolsynthese (13) zugefahren und dort in die Produkte Kohlenwasserstoffe bzw. Methanol umgewandelt wird, zu einem Produkt (18, 20, 21, 22) synthetisiert und gleichzeitig abgekühlt wird (13) und in einer Produktkühlung (15) unter Abscheidung von Wasser gekühlt wird,
**dadurch gekennzeichnet, dass**
bei der Gas- (11) und Produktkühlung (15) innerhalb des Wiederaufbereitungsverfahrens anfallendes Wasser (12) gemeinsam mit externem Wasser (3) für die Kühlung (11) des gebildeten Syntheserohgases und des Syntheseprozesses (13) eingesetzt wird, um das Wasser (3) dabei zu verdampfen und das dampfförmige Wasser (3) in derElektrolyse (4) in Wasserstoff (6) und Sauerstoff (7) aufzuspalten.

## Claims

1. A method for reprocessing the combustion products carbon dioxide (2) and water (3) with electrical energy which has been produced primarily with the aid of regenerative energy sources, but not with the aid of fossil fuels, being coupled in, into renewable synthetic combustion and transportation fuels (18, 20, 21, 22),
wherein an electrolysis (4) of water (3), preferably steam, is operated, the hydrogen (6) formed is mixed with carbon dioxide (2) up to a molar ratio of 3.5 to 1 to form a carbon dioxide/hydrogen mixture (8), the carbon dioxide/hydrogen mixture (8) is preheated in a high-temperature recuperator (9) and then is heated to 800°C to 5000°C in an electrically heated device (5) or an electric plasma generator (5),
the raw synthesis gas which thus forms is used in recuperative manner for preheating the carbon dioxide/hydrogen mixture, then is cooled directly in a gas cooling stage (11) with water being separated off, wherein a synthesis gas is formed which further is supplied to a Fischer-Tropsch synthesis or methanol synthesis stage (13) and is converted there into the products hydrocarbons or methanol, is synthesised into a product (18, 20, 21, 22) and at the same time is cooled down (13), and is cooled in a product cooling stage (15) with water being separated off,
**characterised in that**
water (12) produced within the reprocessing process is used in the gas (11) and product cooling stage (15) together with external water (3) for the cooling (11) of the raw synthesis gas formed and of the synthesis process (13), in order to evaporate the water (3) in so doing and to split the vaporous water (3) into hydrogen (6) and oxygen (7) in the electrolysis stage (4).

## Revendications

1. Procédé pour recycler les produits de combustion dioxyde de carbone (2) et eau (3) en combustibles et carburants synthétiques renouvelables (18, 20, 21, 22) en utilisant de l'énergie électrique qui a été obtenue prioritairement à l'aide de supports d'énergie renouvelable, mais pas à l'aide de combustibles fossiles,
une électrolyse (4) de l'eau (3), de préférence de vapeur d'eau, étant réalisée, l'hydrogène (6) formé étant mélangé avec du dioxyde de carbone (2) pour obtenir un mélange dioxyde de carbone / hydrogène avec un rapport molaire de 1 à 3,5, le mélange dioxyde de carbone / hydrogène (8) étant préchauffé dans un récupérateur à haute température (9) puis chauffé entre 800 °C et 5 000 °C dans un dispositif chauffé électriquement ou dans un générateur de plasma électrique (5),
le gaz de synthèse brut ainsi formé étant utilisé par récupération pour préchauffer le mélange dioxyde de carbone / hydrogène, puis étant refroidi directement dans un refroidisseur de gaz (11) avec séparation de l'eau, un gaz de synthèse se formant lequel est ensuite conduit à une synthèse Fischer-Tropsch ou une synthèse de méthanol (13) où il est transformé en les produits hydrocarbures ou méthanol, synthétisé en un produit (18, 20, 21, 22) et en même temps refroidi (13), et refroidi dans un refroidisseur de produit (15) avec séparation de l'eau,
**caractérisé en ce que**
l'eau recueillie (12) durant le procédé de recyclage dans le refroidisseur de gaz (11) et le refroidisseur de produit (15) est employée avec de l'eau externe (3) pour le refroidissement (11) du gaz de synthèse brut formé et du procédé de synthèse (13), pour vaporiser l'eau (3) et pour fractionner l'eau sous forme vapeur (3) dans l'électrolyse (4) en hydrogène (6) et en oxygène (7).
